# EUROPEAN PATENT SPECIFICATION

(11) **EP 2 197 411 B1**
(45) Date of publication and mention of the grant of the patent: **01.08.2018**
(21) Application number: 08831377.0
(22) Date of filing: 17.09.2008
(51) Int. Cl.: A61K 8/19, A61K 8/58, A61K 8/89, C08K 3/38, C08L 83/04, C08L 83/06

(54) **BORON NITRIDE-CONTAINING SILICONE GEL COMPOSITION**
BORNITRIDHALTIGE SILICONGELZUSAMMENSETZUNG
COMPOSITION DE GEL DE SILICONE CONTENANT DU NITRURE DE BORE

(30) Priority: 20.09.2007 US 903109
(43) Date of publication of application: 23.06.2010
(73) Proprietor: Momentive Performance Materials Inc., Waterford, NY 12188 (US)
(72) Inventor: WAHL, Roy Uwe, Rojas, Teaneck NJ 07666 (US); DUFFY, Janet, Lynn, Wappingers Falls, NY 12590 (US)
(74) Representative: Laufhütte, Dieter
(86) International application number: PCT/US2008/010805
(87) International publication number: WO 2009/038712

(56) References cited:
- WO-A-03/004567
- WO-A-2004/103302
- WO-A-2004/112744
- WO-A-2005/117813
- WO-A-2007/148293
- DE-A1-102005 026 035
- JP-A- 2004 099 458
- JP-A- 2005 200 369
- US-A1- 2005 048 016

## Description

### BACKGROUND OF THE INVENTION

This invention relates to silicone based compositions and, more particularly, to stable boron nitride-containing silicone gel compositions useful, *inter alia*, in the formulation of personal care products.

Silicones have been used for many years in such diverse industrial applications as electronics, agriculture, home- and household care products as well as personal care products and numerous others. While many of these products are in the form of liquids, silicone gels have gained increasing commercial importance, e.g., for their ability to improve the sensory properties, primarily touch and feel, of cosmetics and other kinds of personal care products. Silicone gels offer enhanced functionality to formulators of personal care products, e.g., the capability for augmenting the effect of trisiloxanes and alkytrisiloxanes in the spreading of cosmetic compositions containing such materials .

Boron nitride powders are also known for use in personal care products to which they impart a number of advantageous properties such as softness, adherence, coverage and a natural look due to their low reflectance compared with other materials that have been used for these purposes such as titanium dioxide. An example of such composition is disclosed in JP 2004 099458 A.

While particulate boron nitride comes in several distinct crystalline morphologies, predominantly graphitic (also referred to as hexagonal) forms of boron nitrides have generally been preferred for cosmetic applications where they provide improved slip and/or other properties to these products. However a drawback to the wider acceptance and use of boron nitride in cosmetics and other personal care products, particularly boron nitride of predominantly graphitic crystalline morphology, has been its tendency to result in a gritty appearance and/or a tactile sensation in formulations containing silicone gels. Such tendency is undesirable both for reasons of appearance (the visibility of discrete particles of boron nitride powder in a cosmetic product is a strong consumer negative) and functionality (visible particles, or clumps of particles, of boron nitride are less effective than homogeneously distributed boron nitride particles in forming a smooth and uniform film, an essential prerequisite to obtaining good product coverage.

### SUMMARY OF THE INVENTION

In accordance with the present invention, there is provided a method, according to claim 1, to prepare a stable boron nitride powder-containing silicone gel composition which comprises:
a) at least one silicone gel;
b) at least one boron nitride powder;
c) a static charge-dissipating amount of at least one silicone oil; and,
d) optionally, at least one component selected from the group consisting of other silicone, surfactant, emulsifier, solvent, emollient, moisturizer, humectant, pigment, colorant, fragrance, preservative, antioxidant, biocide, biostat, antiperspirant, exfoliant, hormone, enzyme, medicinal, vitamin substance, hydroxy acid, retinal, retinol derivative, niacinamide, skin lightening agent, salt, electrolyte, alcohol, polyol, ester scattering and/or absorbing agent for ultraviolet radiation, botanical extract, peptide, protein, organic oil, gum, saccharide, oligosaccharide, polysaccharide, wax, film former, thickening agent, particulate filler, plasticizer, humectant, sensory enhancer, resin and optically active particle.

The foregoing stable boron nitride powder-containing silicone gel composition possesses several characteristics which make it ideally suited for use in personal care products to be applied to the skin and/or hair, in particular, a more favorable sensory perception manifested as enhanced properties of touch and feel, e.g., a "silky" quality, and greater hiding power for blemishes, fine lines and wrinkles. In addition, for the manufacturer of personal care products, the silicone gel composition of the invention offers greater ease and convenience in the preparation of fully formulated products, reduces the risk of dusting resulting from the handling of boron nitride powder and eliminates the need to maintain storage and inventory of the separate components of the silicon gel composition.

The term "stable" as it applies to the silicone composition of the invention shall be understood to mean such a composition exhibiting substantially no settling out or separation of any of its components over its expected shelf life, e.g., a period of from 3 months to 2 years depending on the specific formulation of a composition.

As used herein, the expression "silicone gel" refers to any silicone-containing material that increases in volume upon contact with a swelling liquid wherein the swelling liquid diffuses into the silicone-containing material.

The expression "silicone oil" as used herein shall be understood to be synonymous with "silicone fluid."

Other than in the working examples or where otherwise indicated, all numbers expressing amounts of materials, reaction conditions, time durations, quantified properties of materials, and so forth, stated in the specification and claims are to be understood as being modified in all instances by the term "about."

It will also be understood that any numerical range recited herein is intended to include all sub-ranges within that range and any combination of the various endpoints of such ranges or subranges.

It will be further understood that any compound, material or substance which is expressly or implicitly disclosed in the specification and/or recited in a claim as belonging to a group of structurally, compositionally and/or functionally related compounds, materials or substances includes individual representatives of the group and all combinations thereof.

### DETAILED DESCRIPTION OF THE INVENTION

### Silicone Gel (a)

Essentially any silicone gel which is suitable for use in the formulation of personal care products can be utilized in the preparation of the boron nitride-containing silicone gel composition of the invention. Preferred silicone gels include the following:
(i) a gel formed from a silicone and a hydrosilylation compatible solvent wherein said silicone is prepared by the hydrosilylation of a linear alkenyl polyorganosiloxane and a hydride resin;
(ii) a gel formed as a reaction product of an epoxy functional hydrido-siloxane said reaction product being formed in an epoxy-gel formation compatible solvent;
(iii) a gel formed from a silicone and a hydrosilylation compatible solvent wherein said silicone is prepared by the hydrosilylation of a linear hydrogen polyorganosiloxane and an alkenyl resin;
(iv) a gel formed from a silicone and a hydrosilylation compatible solvent wherein said silicone is prepared by the hydrosilylation of a linear hydrogen polyorganosiloxane and a linear alkenyl polyorganosiloxane;
(v) a gel formed from a silicone and hydrosilylation compatible solvent wherein said silicone is prepared by the hydrosilylation of a hydrogen polyorganosiloxane resin and an alkenyl polyorganosiloxane resin;
(vi) a gel formed from a silicone and a hydrosilylation compatible solvent wherein said silicone is prepared by the hydrosilylation of a linear hydrogen organopolysiloxane having two or more hydride functionalities per molecule and an α, ω reactive organic molecule possessing two or more reactive functionalities per molecule;
(vii) a gel formed as a reaction product of a vinyl functional hydrido-siloxane in a hydrosilylation compatible solvent; and,
(viii) a gel formed from a polyacrylate siloxane copolymer network swollen in an aqueous and/or non-aqueous swelling liquid.

The following specific silicone gels are preferred.

### Silicone Gel (i)

In one embodiment, silicone gel (i) comprises:
(A) a first silicone formed by the hydrosilylation product of
   (1) a linear alkenyl polyorganosiloxane having the formula:

      M^{vi}ₐDₓD^{vi}_{y}M₂₋ₐ

      where the subscript x is a number greater than 10, the subscript y is a number ranging from zero to about 20, the subscript a is a number ranging from 0 to 2, subject to the limitation that a+y is within the range of from 1 to about 20. with M^{vi} defined as:

      R¹R²R³SiO_{1/2}

      where R¹ is a monovalent unsaturated hydrocarbon radical having from two to ten carbon atoms, and R² and R³ are each independently one to forty carbon atom monovalent hydrocarbon radicals, with D defined as:

      R⁴R⁵SiO_{2/2}

      where R⁴ and R⁵ are each independently one to forty carbon atom monovalent hydrocarbon radicals, with D^{vi} defined as:

      D^{vi}=R⁶R⁷SiO_{2/2}

      where R⁶ is a monovalent unsaturated hydrocarbon radical having from two to ten carbon atoms, and R⁷ is independently a one to forty carbon atom monovalent hydrocarbon radical with M defined as

      M=R⁸R⁹R¹⁰SiO_{1/2}

      with R⁸, R⁹, and R¹⁰ each independently a one to forty carbon atom monovalent hydrocarbon radical; and,
   (2) a hydride resin having the formula:

      (M^{H}_{w}Q_{z})ⱼ

      where Q has the formula SiO_{4/2} and with M^{H} defined as

      H_{b}R¹¹₃-bSiO_{1/2}

      where R¹¹ is a one to forty carbon atom monovalent hydrocarbon radical where the subscript b is a number ranging from 1 to 3, with the subscripts w and z having a ratio of 0.5 to 4.0 respectively and the subscript j ranges from about 2.0 to about 100; wherein said hydrosilylation is conducted in the presence of
   (3) a hydrosilylation compatible solvent preferably a silicone having a viscosity below about 1,000 centistokes at 25.degree. C. or a hydrosilylation compatible lipohilic phase (hereinafter also referred to as a hydrosilylation compatible solvent), thereby forming a gel; and
(B) a lipophilic phase or a silicone having a viscosity below about 1,000 centistokes at 25.degree. C. (hereinafter also referred to as dispersant medium or media) wherein said hydrosilylation product is slurried in said lipophilic phase or said silicone and subjected to mixing with said lipophilic phase or said silicone; producing thereby a uniform mixture comprising said lipophilic phase or said silicone and said hydrosilylation product whereby said uniform mixture has a viscosity ranging from 500 to 500,000 centistokes at 25 degree C.

The silicone having a viscosity below about 1,000 centistokes at 25.degree. C. is preferably selected from the group consisting of cyclic silicones having the formula:

D_{f}

where the subscript f is an integer ranging from about three to about 6 with D defined as:

R⁴R⁵SiO_{2/2}

where R⁴ and R⁵ are each independently one to forty carbon atom monovalent hydrocarbon radicals and
linear silicones having the formula:

M'D'ᵢM'

where D' is defined as:

R⁴R⁵SiO_{2/2}

where R⁴ and R⁵ are each independently one to forty carbon atom monovalent hydrocarbon radicals and M' has the formula:

R¹²R¹³R¹⁴SiO_{1/2}

where R¹², R¹³ and R¹⁴ are each independently one to forty carbon atom monovalent hydrocarbon radicals.

### Silicone Gel (ii)

In one embodiment, silicone gel (ii) comprises the reaction products of an epoxy functional hydrido-siloxane molecule having the following formula:

M_{α}M^{H}_{β}M^{E}_{χ}D_{δ}DH_{ε}D^{E}_{ϕ}T_{γ}T^{H}_{η}T^{E}_{τ}

Q_{κ} where

M=R^{1'}R^{2'}R^{3'}SiO_{1/2} ;

M^{H}=R^{4'}R^{5'}HSiO_{1/2} ;

M^{E}=R^{6'}R^{7'}R^{E}SiO_{1/2};

D=R^{8'}R^{9'}SiO_{2/2};

D^{H}=R^{10'}HsiO_{2/2};

D^{E}=R^{11'}R^{E}SiO_{2/2};

T=R^{12'}SiO_{3/2};

T^{H}=HsiO_{3/2};

T^{E}=R^{E}SiO_{3/2};

and

Q=SiO_{4/2};

where R1', R2', R3', R8', and R12' are independently monovalent hydrocarbon radicals having from one to sixty carbon atoms; R4', R5' and R10' are independently monovalent hydrocarbon radicals having from one to sixty carbon atoms or hydrogen; R6', R7', R11' are independently monovalent hydrocarbon radicals having from one to sixty carbon atoms or RE; each RE is independently a monovalent hydrocarbon radical containing one or more oxirane moieties having from one to sixty carbon atoms; the stoichiometric subscripts α, β, χ, δ, ε, ϕ, γ, η, τ, and κ are either zero or positive subject to the following limitations: α+β+χ>1; β+εη>1; χ+ϕ+τ>1; β+ε+η≥χ+ϕ+τ; and when δ+ε+ϕ+γ+η+τ+κ=0, α+β+χ=2.

The reaction product of an epoxy functional hydrido siloxane molecule is preferably prepared in an epoxy gel formation medium selected from a lipophilic phase or a silicone fluid selected from the group consisting of cyclic silicones having the formula:

D_{f}

where the subscript f is an integer ranging from about three to about 6 with D defined as:

R⁴R⁵SiO_{2/2}

where R⁴ and R⁵ are each independently one to forty carbon atom monovalent hydrocarbon radicals and
linear silicones having the formula:

M'D'ᵢM'

where D' is defined as:

R⁴R⁵SiO_{2/2}

where R⁴ and R⁵ are each independently one to forty carbon atom monovalent hydrocarbon radicals and M' has the formula:

R¹²R¹³R¹⁴SiO_{1/2}

where R¹², R¹³ and R¹⁴ are each independently one to forty carbon atom monovalent hydrocarbon radicals.

Once prepared, silicone gel (ii) may be slurried and mixed in a dispersant medium selected from a lipophilic phase or a silicone selected from the group consisting of cyclic silicones having the formula

D_{f}

where the subscript f is an integer ranging from about three to about 6 with D defined as

R⁴R⁵SiO_{2/2}

where R⁴ and R⁵ are each independently one to forty carbon atom monovalent hydrocarbon radicals and
linear silicones having the formula

M'D'ᵢM'

where D' is defined as

R⁴R⁵SiO_{2/2}

where R⁴ and R⁵ are each independently one to forty carbon atom monovalent hydrocarbon radicals and M' has the formula

R¹²R¹³R¹⁴SiO_{1/2}

where R¹², R¹³ and R¹⁴ are each independently one to forty carbon atom monovalent hydrocarbon radicals.

### Silicone Gel (iii)

In one embodiment, silicone gel (iii) comprising:
(A) a silicone formed by the hydrosilylation product of
   (1) a linear hydrogen polyorganosiloxane having the formula:

      M^{H}ₐDₓD^{H}_{y}M₂₋ₐ

      where the subscript x is a number greater than 10, the subscript y is a number ranging from zero to about 20, the subscript a is a number ranging from 0 to 2, subject to the limitation that a+y is within the range of from 1 to about 20, with M^{H} defined as:

      R¹R²R³SiO_{1/2}

      where R¹ is hydrogen, R² and R³ are each independently one to forty carbon atom monovalent hydrocarbon radicals, with D defined as:

      R⁴R⁵SiO_{2/2}

      where R⁴ and R⁵ are each independently one to forty carbon atom monovalent hydrocarbon radicals, with D^{H} defined as:

      D^{H}=R⁶R⁷SiO_{2/2}

      where R⁶ is hydrogen and R⁷ is independently a one to forty carbon atom monovalent hydrocarbon radical with M defined as

      M=R⁸R⁹R¹⁰SiO_{1/2}

      with R⁸, R⁹, and R¹⁰ each independently a one to forty carbon atom monovalent hydrocarbon radical; and
   (2) an alkenyl resin having the formula:

      (M^{vi}._{w}Q_{z})ⱼ

      where Q has the formula SiO_{4/2} and with M^{vi} defined as

      R¹¹_{b}R¹²₃-bSiO_{1/2}

      where R¹¹ is a monovalent unsaturated hydrocarbon radical having from two to ten carbon atoms, R¹² is a one to forty carbon atom monovalent hydrocarbon radical where the subscript b is a number ranging from 1 to 3, with the subscripts w and z having a ratio of 0.5 to 4.0 respectively and the subscript j ranges from about 2.0 to about 100; wherein said hydrosilylation is conducted in the presence of
   (3) a hydrosilylation compatible solvent preferably a silicone having a viscosity below about 1,000 centistokes at 25°C or a hydrosilylation compatible lipohilic phase (hereinafter also referred to as hydrosilylation compatible solvent), thereby forming a gel; and,
(B) a lipophilic phase or a silicone having a viscosity below about 1,000 centistokes at 25.degree. C. (hereinafter also referred to as dispersant medium or media) wherein said hydrosilylation product is slurried in said lipophilic phase or said silicone and subjected to mixing with said lipophilic phase or said silicone; producing thereby a uniform mixture comprising said lipophilic phase or said silicone and said hydrosilylation product whereby said uniform mixture has a viscosity ranging from 500 to 500,000 centistokes at 25°C.

### Silicone Gel (iv)

In one embodiment, silicone gel (iv) comprises the hydrosilylation reaction product of a linear alkenyl organopolysiloxane (as previously defined) having two or more alkenyl functionalities per molecule as above with a linear hydrogen organopolysiloxane (as previously defined) having two or more hydrogen functionalities per molecule prepared as above in the presence of a hydrosilylation compatible solvent or silicone, D_{f} and/or M'D'ᵢM' where D_{f} and M'D'ᵢM' are as previously defined. The gel as prepared may then be slurried with a lipophilic phase or a silicone having a viscosity below about 1,000 centistokes at 25°C (hereinafter also referred to as dispersant medium or media) wherein said hydrosilylation product is slurried in said lipophilic phase or said silicone and subjected to mixing with said lipophilic phase or said silicone; producing thereby a uniform mixture comprising said lipophilic phase or said silicone and said hydrosilylation product whereby said uniform mixture has a viscosity ranging from 500 to 500,000 centistokes at 25°C.

### Silicone Gel (v)

In one embodiment, silicone gel (v) comprises the hydrosilylation reaction product of an alkenyl organopolysiloxane resin having two or more alkenyl functionalities per molecule having the formula

M^{vi}_{c}D_{d}D^{vi}ₑT_{g}T^{vi}ₖM_{2-c}M"_{g}+-k

with a hydrogen organopolysiloxane resin having two or more hydrogen functionalities per molecule having the formula

M^{H}ₙDₚD^{H}ᵣTₛT^{H}ᵤMᵥM"ₛ+u

where all the terms are as previously defined with
T=R¹⁶SiO_{3/2} where R¹⁶ is a one to forty carbon atom monovalent hydrocarbon radicals;
T^{vi}=R¹⁷SiO_{3/2} where R¹⁷ is a monovalent unsaturated hydrocarbon radical having from two to forty carbon atoms;
T^{H}=HSiO_{3/2};
M" is independently M^{H}, M^{vi} or M and the subscripts c, d, e, g, k, n, p, r, s, u, and v are either zero or positive subject to the limitations that g+k+s+u is ≥1 ; c+e+k ≥2 and n+r+u≥2; prepared in a hydrosilylation compatible solvent and slurried in a lipophilic phase or a silicone having a viscosity below about 1,000 centistokes at 25°C (hereinafter also referred to as dispersant medium or media) wherein said hydrosilylation product is slurried in said lipophilic phase or said silicone and subjected to mixing with said lipophilic phase or said silicone; producing thereby a uniform mixture comprising said lipophilic phase or said silicone and said hydrosilylation product whereby said uniform mixture has a viscosity ranging from 500 to 500;000 centistokes at 25°C.

### Silicone Gel (vi)

In one embodiment, silicone gel (vi) comprises the reaction product of a linear hydrido organopolysiloxane having two or more hydride functionalities per molecule with an α, ω reactive organic molecule possessing two or more reactive functionalities per molecule in the presence of a lipophilic phase or second silicone, D_{f} and/or M'D'ᵢM' where D_{f} and M'D'ᵢM' are as previously defined. The reactive functionalities of the α, ω reactive organic molecule possessing two or more functionalities per molecule are selected from the group of organic functional groups consisting of olefins, acetylenes, vinylethers, acrylates or acrylate esters (eg CH2=CHCOOROCOCH=CH2), and alcohols and the like. Thus the α, ω reactive organic molecule possessing two or more functionalities per molecule subtends a large group of organic molecules that includes α, ω-di-olefins, α, ω-olefins possessing a polyolefinic functionality, α, ω-di-acetylenes, α, ω-di-acetylenes possessing a polyacetylenic functionality, including side chain substituted variations where the side chains possess reactive functionality as herein defined. This gel is prepared in a hydrosilylation compatible solvent and slurried in a lipophilic phase or a silicone having a viscosity below about 1,000 centistokes at 25°C (hereinafter also referred to as dispersant medium or media) wherein said hydrosilylation product is slurried in said lipophilic phase or said silicone and subjected to mixing with said lipophilic phase or said silicone; producing thereby a uniform mixture comprising said lipophilic phase or said silicone and said hydrosilylation product whereby said uniform mixture has a viscosity ranging from 500 to 500,000 centistokes at 25°C.

### Silicone Gel (vii)

In one embodiment, silicone gel (vii) comprises the reaction products of a vinyl functional hydrido-siloxane molecule having the following formula:

M_{a'}M^{vi}_{b'}M^{H}_{c'}D_{d'}D^{vi}_{e'}D^{H}_{f}'T_{g'}T^{vi}_{h'}T^{H}_{i'}Q_{j'}

where all the terms are as previously defined and the subscripts a', b', c', d', e', f, g', h', i' and j' are either 0 or a positive integer for well defined molecular species subject to the limitation b'+e'+h' is greater than or equal to one and further subject to the limitation that c'+f+i' is greater than or equal to one. Mixtures of compounds where individual molecular species possess the formula, Mₐ'M^{vi}_{b}'M^{H}_{c}'D_{d}'D^{vi}ₑ'D^{H}_{f}- 'T_{g}'T^{vi}ₕ'T^{H}ᵢ'Qⱼ', will analyze for non-integral values of the subscripts because of the fact that it is a mixture and not a pure compound. Thus for mixtures of compounds possessing the formula. M_{a'}M^{vi}_{b'}M^{H}_{c'}D_{d'}D^{vi}_{e'}D^{H}_{f'}T_{g'}T^{vi}_{h'}T^{H}_{i'}Q_{j'}, the subscripts a', b', c', d', e', f', g', h', i' and j' will be zero or positive. Compounds possessing the formula M_{a'}M^{vi}_{b'}M^{H}- _{c'}D_{d'}D^{vi}_{e'}D^{H}_{f'}T_{g'}T^{vi}_{h'}T^{H}_{i'}Q_{j'} may be prepared by the procedures and methods disclosed in U.S. Pat. Nos. 5,698,654; 5,753,751; and 5,965,683. These materials may be reacted with the silicone precursors to the class II silicone gels previously defined herein or they may self-reacted in the presence of a noble metal hydrosilylation catalyst as is known in the art. These materials are prepared in a hydrosilyation compatible solvent and slurried in a lipophilic phase or a silicone having a viscosity below about 1,000 centistokes at 25.degree. C. (hereinafter also referred to as dispersant medium or media) wherein said hydrosilylation product is slurried in said lipophilic phase or said silicone and subjected to mixing with said lipophilic phase or said silicone; producing thereby a uniform mixture comprising said lipophilic phase or said silicone and said hydrosilylation product whereby said uniform mixture has a viscosity ranging from 500 to 500,000 centistokes at 25°C.

Many types of noble metal catalysts for hydrosilylation (or SiH olefin addition reaction) are known and such noble metal catalysts may be used for the preparative reactions involved in making the compositions of the present invention. The most preferred noble metals are those of the platinum group metals, specifically rhodium and platinum. When optical clarity of the resulting addition product is required the preferred catalysts are those catalysts that are compounds that are soluble in the reaction mixture. One such platinum compound can be selected from those having the formula (PtCl₂Olefin) and H(PtCl₃Olefin) as described in U.S. Pat. No. 3,159,601. The olefin shown in the previous two catalyst compound formulas can be almost any type of olefin but is preferably an alkenylene having from 2 to 8 carbon atoms, a cycloalkenylene have from 5 to 7 carbon atoms or styrene. Specific olefins utilizable in the above formulas are ethylene, propylene, the various isomers of butylene, octylene, cyclopentene, cyclohexene, or cycloheptene.

A further platinum-containing material usable in the compositions of the present invention is the cyclopropane complex of platinum chloride described in U.S. Pat. No. 3,159,662.

Further the platinum-containing material can be a complex formed from chloroplatinic acid with up to 2 moles per gram of platinum of a member selected from the class consisting of alcohols, ethers, aldehydes and mixtures of the above as described in U.S. Pat. No. 3,220.

The catalysts are described in U.S. Pat. Nos. 3,715,334; 3,775,452; and 3,814,730 to Karstedt. Additional background concerning the art may be found at J. L. Spier, "Homogeneous Catalysis of Hydrosilation by Transition Metals, in Advances in Organometallic Chemistry, volume 17, pages 407 through 447, F. G. A. Stone and R. West editors, published by the Academic Press (New York, 1979). Persons skilled in the art can easily determine an effective amount of noble metal or platinum catalyst. Generally, an effective amount ranges from about 0.1 to 50 parts per million of the total organopolysiloxane composition.

The gels of the present invention are prepared either in a hydrosilylation compatible medium or solvent or an epoxy-gel formation compatible medium or solvent depending on the chemical nature of the gel being prepared. Both classes of preparation media include silicone solvents, preferably a silicone selected from the group consisting of cyclic silicones having the formula

D_{f}

where the subscript f is an integer ranging from about three to about 6 with D defined as

R⁴R⁵SiO_{2/2}

where R⁴ and R⁵ are each independently one to forty carbon atom monovalent hydrocarbon radicals and
linear silicones having the formula

M'D'ᵢM'

where D' is defined as

R⁴R⁵SiO_{2/2}

where R⁴ and R⁵ are each independently one to forty carbon atom monovalent hydrocarbon radicals and M' has the formula

R¹²R¹³R¹⁴SiO_{1/2}

where R¹², R¹³ and R¹⁴ are each independently one to forty carbon atom monovalent hydrocarbon radicals.

The hydrosilylation compatible medium or solvent are selected from the group consisting of silicones and substituted silicones including: silicone oils of the desired viscosity from D₄ to 10,000 cps oils; polyethersilicone copolymers where the polyethers vary from 200 to 3000 molecular weight and may consist of alkylene oxide chains based on one, two or more types of monomer units such as ethylene oxide, propylene oxide or butylene oxide and may be attached to the silicone with 1 to six carbon chain, or through an silicone oxygen bond; polyester silicone copolymers; alkyl, aromatic or alkylaromatic substituted siloxanes; alkoxy substituted siloxanes including: substituted methoxy, ethoxy, propoxy, octyloxy, dodecanoxy, cetyryloxy or isostearyloxy siloxanes or other organically substituted siloxanes or siloxanes containing multiple organic substituents that are compatible with hydrosilylation reactions; hydro carbon solvents including: tetradecane, isododecane, isohexadecane, mineral oil, hydrogenate polydecene, apricot oil; ester solvents including: isopropyl myristate, diisopropyl adipate, isodecyl neopentanoate; ethers including: PPG-14 butyl ether, PPG 3 myristyl ether, ethoxylated alkylphenols; glyceryl esters of fatty acids including: sunflower oils, caprylic/capric triglyceride, C.sub.10-18 triglyceride; fatty acid glycerides including: glyceryl stearate, glyceryl dioleate; non-volatile fluorinated oil including: fluorinated silicones and fluorinated esters; aromatic solvents including; benzene, toluene and alkylbenzenes; and alcohols including: isopropanol, octanol, dodecanol, hexadecanol, cetearyl alcohol, isostearyl alcohol, myristyl alcohol.

The epoxy gel formation compatible medium or solvent is primarily defined by solvent inertness and is preferably selected from the group of silicone solvents D_{f} and M'D'ᵢM' as previously defined and hydrocarbon solvents selected from the group consisting of paraffinic, iso-paraffinic, aromatic and alkyl aromatic solvents.

### Silicone Gel (viii)

Additionally useful silicone gels (a) herein are those comprising the polyacrylate siloxane copolymer network of pending U.S. patent application Serial No. 11/742,190, filed April 30, 2007, swollen in an aqueous and/or non-aqueous swelling liquid. This polyacrylate siloxane copolymer network comprises the reaction product of:

MₐM^{H}_{b-h-k}M^{PE}ₕM^{E}ₖD_{c}D^{H}_{d-i-l}D^{PE}ᵢD^{E}ₗTₑT^{H}_{f-j-m}T^{PE}ⱼT^{E}ₘQ_{g}

and a stoichiometric or super-stoichiometric quantity of acrylate where

M = R¹R²R³SiO_{1/2};

M^{H} = R⁴R⁵HSiO_{1/2};

M^{PE} = R⁴R⁵(-CH₂CH(R⁹)(R¹⁰)ₙO(R¹¹)ₒ(C₂H₄O)ₚ(C₃H₆O)_{q}(C₄H₈O)ᵣR¹²)SiO_{1/2};

M^{E} = R⁴R⁵(-R¹⁷R¹⁸C-CR¹⁶QₛQₜR¹⁵(COC)R¹³R¹⁴)SiO_{1/2}

D = R⁶R⁷SiO_{2/2};

and

D^{H} = R⁸HSiO_{2/2}

D^{PE} = R⁸SiO_{2/2}

D^{E} = R⁸(-R¹⁷R¹⁸C-CR¹⁶QₛQₜR¹⁵(COC)R¹³R¹⁴)SiO_{2/2}.

T = R¹⁹SiO_{3/2};

T^{H} = HSiO_{3/2};

T^{PE} = (-CH₂CH(R⁹)(R¹⁰)ₙO(R¹¹)ₒ(C₂H₄O)ₚ(C₃H₆O)_{q}(C₄H₈O)ᵣR¹²)SiO_{3/2};

T^{E} = (-R¹⁷R¹⁸C-CR¹⁶QₛQₜR¹⁵(COC)R¹³R¹⁴)SiO_{3/2};

and

Q = SiO_{4/2};

where R¹, R², R³, R⁴, R⁵, R⁶, R⁷, R⁸ and R¹⁹ are each independently selected from the group of monovalent hydrocarbon radicals having from 1 to 60 carbon atoms;
R⁹ is H or a 1 to 6 carbon atom alkyl group; R¹⁰ is a divalent alkyl radical of 1 to 6 carbons;
R¹¹ is selected from the group of divalent radicals consisting of -C₂H₄O-, -C₃H₆O-, and -C₄H₈O-; R¹² is H, a monofunctional hydrocarbon radical of 1 to 6 carbons, or acetyl; R¹³, R¹⁴, R¹⁵, R¹⁶, R¹⁷ and R¹⁸ are each independently selected from the group of hydrogen and monovalent hydrocarbon radicals having from one to sixty carbon atoms, Qₜ is a di- or trivalent hydrocarbon radical having from one to sixty carbon atoms, Qₛ is a divalent hydrocarbon radical having from one to sixty carbon atoms subject to the limitation that when Qₜ is trivalent R¹⁴ is absent and Qₛ forms a bond with the carbon bearing R¹³ where R¹⁶ and R¹⁸ may be either cis- or trans- to each other;
the subscript a may be zero or positive subject to the limitation that when the subscript a is zero, b must be positive;
the subscript b may be zero or positive subject to the limitation that when b is zero, the subscript a must be positive;
the subscript c is positive and has a value ranging from about 5 to about 1,000;
the subscript d is positive and has a value ranging from about 3 to about 400;
the subscript e is zero or positive and has a value ranging from 0 to about 50;
the subscript f is zero or positive and has a value ranging from 0 to about 30;
the subscript g is zero or positive and has a value ranging from 0 to about 20;
the subscript h is zero or positive and has a value ranging from 0 to about 2 subject to the limitation that the sum of the subscripts h, i and j is positive;
the subscript i is zero or positive and has a value ranging from 0 to about 200 subject to the limitation that the sum of the subscripts h, i and j is positive;
the subscript j is zero or positive and has a value ranging from 0 to about 30 subject to the limitation that the sum of the subscripts h, i and j is positive;
the subscript k is zero or positive and has a value ranging from 0 to about 2 subject to the limitation that the sum of the subscripts k, 1 and m is positive;
the subscript 1 is zero or positive and has a value ranging from 0 to about 200 subject to the limitation that the sum of the subscripts k, 1 and m is positive;
the subscript m is zero or positive and has a value ranging from 0 to about 30 subject to the limitation that the sum of the subscripts k, l and m is positive;
the subscript n is zero or one;
the subscript o is zero or one;
the subscript p is zero or positive and has a value ranging from 0 to about 100 subject to the limitation that (p + q + r) > 0;
the subscript q is zero or positive and has a value ranging from 0 to about 100 subject to the limitation that (p + q + r) > 0;
the subscript r is zero or positive and has a value ranging from 0 to about 100 subject to the limitation that (p + q + r) > 0;
the subscript s is zero or one; and,
the subscript t is zero or one;
   in the presence of a free radical initiator.

Water (or a water equivalent such as a non-aqueous hydroxylic solvent), siloxane, linear or cyclic, or lipophilic fluid (oil swelling agent, oil swellable) may be used as the swelling agent. Lipophilic fluids suitable for use as the fluid component of the composition of the present invention are those compounds or mixtures of two or more compounds that are in the liquid state at or near room temperature, for example, from about 20°C about 50°C, and about one atmosphere pressure, and include, for example, silicone fluids, hydrocarbon fluids, esters, alcohols, fatty alcohols, glycols and organic oils. In a preferred embodiment, the fluid component of the composition of the present invention exhibits a viscosity of below about 1,000 cSt, preferably below about 500 cSt, more preferably below about 250 cSt, and most preferably below 100 cSt, at 25 °C.

For the compositions in accordance with the present invention, the total amount of silicone gel (a) ranges from 50 to 80 weight percent and more preferably from 50 to 75 weight percent, the balance of the compositions being made up of boron nitride powder (b) and silicone oil (b).

### Boron Nitride Powder (b)

Boron nitride powder (b) employed in the composition of the invention can possess any of the boron nitride crystalline morphologies, e.g., hexagonal and cubic morphologies. Among the hexagonal morphologies are turbostratic boron nitride, an early process form of boron nitride powder with small crystal size and less ordered platelets, and graphitic boron nitride powder with larger crystals, as well as quasigraphitic boron nitride powder which is in between both aforementioned morphologies.

Especially advantageous for use herein are any of the following Softouch™ boron nitride powders available from Momentive Performance Materials:

| Designation | Average Particle Size | Predominant Crystal Morphology |
|---|---|---|
| CC6004 | 11.0 | Graphitic |
| CC6064 | 11.5 | Graphitic |
| CC6058 | 11.0 | quasigraphitic |
| CC6059 | 6.0 | quasigraphitic |
| CC6069 | 9.0 | quasigraphitic |
| CCS102 | 15.0 | quasigraphitic |
| CCS402 | 47.0 | quasipraphitic |
| CCX1 | 0.7 | quasigraphitic |
| CC6098 | 2.0 | turbostratic |
| CC6097 | 5.0 | turbostratic |

The amount of boron nitride powder incorporated in the composition of the invention can vary widely depending on the particular formulation involved and its desired functional properties.

In general, the amounts of boron nitride powder in the composition of the invention can range from 0.5 to 40 weight percent, preferably from 5 to 30 weight percent and more preferably from 10 to 20 weight percent, the balance of the composition being made up of silicone gel (a) and silicone oil (c).

### Silicone Oil (c)

Silicone oil (c) can be selected from among any of the known and conventional silicone oils heretofore employed in personal care products.

Representative silicone oils include the volatile linear or cyclic silicone oils such as those having a viscosity ≤8 centistokes (8x10⁻⁶ m^{2/8}), and having, for example from 2 to 7 silicon atoms, these silicones optionally comprising alkyl or alkoxy groups having from 1 to 10 carbon atoms. As volatile silicone oil which can be used in the invention there may be mentioned octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane, dodecamethylpentasiloxane, capryl methicone and mixtures thereof.

Also useful herein are the nonvolatile silicone oils such as polydimethylsiloxanes (PDMS), polydimethylsiloxanes comprising alkyl or alkoxy groups, pendant and/or at the silicone chain end, the alkyl and alkoxy groups each having from 2 to 24 carbon atoms, phenylated silicones such as phenyltrimethicones, phenyldimethicones, phenyltrimethylsiloxydiphenylsiloxanes, diphenyldimethicones, diphenylmethyl-diphenyltrisiloxanes and (2-phenylethyl)trimethylsiloxy-silicates.

Silicone oil (c) must be present in the silicone gel composition of the invention in at least a static charge-dissipating amount, an amount that can be empirically determined for a particular silicone gel-containing composition employing routine experimentation.

In general, the silicone gel-containing composition of the invention can contain from 1.5 to 40 weight percent, preferably from 5 to 30 weight percent, and more preferably from 10 to 20 weight percent, silicone oil (c).

### Optional Component(s) (d)

The silicone gel-containing composition of the invention may also contain one or more optional components such as any of the ingredients of known and conventional personal care products employed in the customary amounts. Among these ingredients may be mentioned other silicone, surfactant, emulsifier, solvent, emollient, moisturizer, humectant, pigment, colorant, fragrance, preservative, antioxidant, biocide, biostat, antiperspirant, exfoliant, hormone, enzyme, medicinal, vitamin substance, hydroxy acid, retinal, retinol derivative, niacinamide, skin lightening agent, salt, electrolyte, alcohol, polyol, ester, scattering and/or absorbing agent for ultraviolet radiation, botanical extract, peptide, protein, organic oil, gum, saccharide, oligosaccharide, polysaccharide, wax, film former, thickening agent, particulate filler, plasticizer, humectant, sensory enhancer, resin and optically active particle.

Personal care products where the stable boron nitride powder-containing silicone gel composition of the present invention may be employed include, but are not limited to, deodorants, antiperspirants, antiperspirant/deodorants, shaving products, skin lotions, moisturizers, toners, bath products, cleansing products, hair care products such as shampoos, conditioners, mousses, styling gels, hair sprays, hair dyes, hair color products, hair bleaches, waving products, hair straighteners, manicure products such as nail polish, nail polish remover, nails creams and lotions, cuticle softeners, protective creams such as sunscreen, insect repellent and anti-aging products, color cosmetics such as lipsticks, foundations, face powders, eye liners, eye shadows, blushes, makeup, mascaras and other personal care formulations where silicone components have been conventionally added, as well as drug delivery systems for topical application of medicinal compositions that are to be applied to the skin.

### EXAMPLE 1

A stable boron nitride powder-containing silicone gel composition in accordance with the invention (Silicone Gel A) was prepared by combining the following ingredients (all from Momentive Performance Materials):

| Ingredient | Description | Wt. Amount, g | Wt. % |
|---|---|---|---|
| Velvesil 125 silicone gel | (crosslinked C30-45 alkyl cetearyl dimethicone in cyclopentasiloxane) | 48.72 | 69.60 |
| Softouch™ CC6097 | boron nitride powder | 12.18 | 17.40 |
| Silsoft™ 034 | silicone oil (caprlyl methicone) | 9.10 | 13.00 |
| | | 70.00 g | 100.00% |

The silicone gel, boron nitride powder and silicone oil were stirred together at 600 rpm with an overhead mixer equipped with a cross-blade to provide a silicone gel composition having a semi-granular or flaky past consistency.

### EXAMPLE 2

Employing the same ingredients as in Example 1, another stable boron nitride powder-containing silicone gel composition was prepared in accordance with the invention (Silicone Gel B). The amounts of each ingredient in the gel were as follows:

| Ingredient | Wt. Amount, g |
|---|---|
| Velvesil 125 silicone gel | 69.6 |
| Softouch™ CC6097 | 17.4 |
| Silsoft™ 034 | 13.0 |
| | 100.0 g |

The gel was prepared by first adding Silsoft™ 034 to Velvesil 125 and thereafter adding Softouch™ CC6097 while stirring for 15 minutes at ambient temperature using an overhead mixer equipped with a 3-bladed paddle at 100 g. scale.

### EXAMPLES 3 AND 4

Foundation compositions containing the same ingredients in substantially the same amounts were prepared by sequentially mixing the ingredients indicated below at 500 rpm and 80°C for 20 minutes (all amounts in weight percent):

| Ingredient | Example 3 | Example 4 |
|---|---|---|
| Velvesil 125 | 29.89 | 40.20 |
| polysilsesquioxane | 10.62 | 10.62 |
| cyclopentasiloxane | 36.63 | 36.63 |
| Penylproplydimethyl-siloxy silicate | 2.71 | 2.71 |
| C30-45 alkyldimethicone | 5.33 | 5.33 |
| Silsoft™ 034 | ----- | 1.92 |
| Silicone Gel A (from Example 1) | 14.82 | ----- |
| SoftouchTM CC6097 | ---- | 2.58 |
| | 100.00 | 99.99 |

Each foundation composition was applied to Lenetta paper and drawn down to provide a 5 mil thickness film. The film obtained with the foundation composition of Example 3 showed no visible agglomeration while agglomeration was cleanly visible in the case of the film obtained with the foundation composition of Example 4. This difference in results can be accounted for by the different manner in which the foundation compositions were made. In the case of the composition of Example 3, some of the silicone gel, all of the boron nitride provider and all of the silicone oil constituting the boron nitride powder-containing silicone gel of the invention were added as a pre-mix (Silicone Gel A of Example 1), whereas in the composition of Example 4, the silicone gel, boron nitride powder and silicone oil were added individually and at intervals separated by the addition of the other ingredients of the composition. Thus, in the manufacture of a personal care product, in order to avoid or reduce the risk of agglomeration, it is preferred to add the silicone gel composition of the invention to the other ingredients in the formulation as a pre-mix rather than individually and in interrupted sequence.

The films obtained from the foundation compositions of Examples 3 and 4 were also evaluated for coverage (L value with the higher value indicating greater coverage) as measured by a Hunter Lab Color Quest 45/0 spectraphotometer. The film made from the composition of Example 3 had an L value of 0.55 while the film made from the composition of Example 4 had an L value of 0.44. These results further demonstrate the benefit of employing the silicone gel composition as a pre-mix rather than as individual ingredients when manufacturing a personal care product.

### EXAMPLE 5

Another stable boron nitride powder-containing silicone gel composition was prepared in accordance with the invention (Silicone Gel C). The amounts of each ingredient in the gel were as follows:

| Ingredient | Description | Wt. Amount, g | Wt. % |
|---|---|---|---|
| Velvesil DM silicone gel | (cetearyl dimethicone crosspolymer in dimethicone) | 71.60 | 71.60 |
| Softouch™ CC6097 | boron nitride powder | 17.90 | 19.90 |
| Silsoft™ 034 | silicone oil (caprlyl methicone) | 10.50 | 10.50 |
| | | 100.00 g | 100.00% |

The silicone gel and silicone oil were stirred together by hand and then mixed with an overhead mixer at 600 rpm for 10 minutes. Thereafter, the boron nitride powder was added and the mixture stirred for another 20 minutes.

Other embodiments of the invention will be apparent to those skilled in the art from a consideration of this specification or practice of the invention disclosed herein. It is intended that the specification and examples be considered as exemplary only, with the true scope of the invention being defined by the following claims.

## Claims

1. A process for preparing a stable boron nitride powder-containing silicone gel personal care composition for application to skin or hair and comprising:
(A) combining
(a) a source of silicone gel(s) containing not more than 10 weight percent thereof of non-silicone gel material(s),
(b) a source of boron nitride powder(s) and
(c) a source of silicone oil(s)
to provide a stable, uniform mixture of (a), (b) and (c), which contains from 50 to 80 weight percent of silicone gel(s), with the balance of the composition being made up of boron nitride powder and silicone oil; and,
(B) combining the stable, uniform mixture of (a), (b) and (c) with at least one component selected from the group consisting of other silicone, surfactant, emulsifier, solvent, emollient, moisturizer, humectant, pigment, colorant, fragrance, preservative, antioxidant, biocide, biostat, antiperspirant, exfoliant, hormone, enzyme, medicinal, vitamin substance, hydroxy acid, retinal, retinol derivative, niacinamide, skin lightening agent, salt, electrolyte, alcohol, polyol, ester scattering and/or absorbing agent for ultraviolet radiation, botanical extract, peptide, protein, organic oil, gum, saccharide, oligosaccharide, polysaccharide, wax, film former, thickening agent, particulate filler, plasticizer, humectant, sensory enhancer, resin and optically active particle.

2. The process of Claim 1 wherein:
a) the source of silicone gel(s) (a) contains at least one member selected from the group consisting of:
(i) a gel formed from a silicone and a hydrosilylation compatible solvent wherein said silicone is prepared by the hydrosilylation of a linear alkenyl polyorganosiloxane and a hydride resin,
(ii) a gel formed as a reaction product of an epoxy functional hydrido-siloxane said reaction product being formed in an epoxy-gel formation-compatible solvent,
(iii) a gel formed from a silicone and a hydrosilylation compatible solvent wherein said silicone is prepared by the hydrosilylation of a linear hydrogen polyorganosiloxane and an alkenyl resin,
(iv) a gel formed from a silicone and a hydrosilylation compatible solvent wherein said silicone is prepared by the hydrosilylation of a linear hydrogen polyorganosiloxane and a linear alkenyl polyorganosiloxane,
(v) a gel formed from a silicone and hydrosilylation compatible solvent wherein said silicone is prepared by the hydrosilylation of a hydrogen polyorganosiloxane resin and an alkenyl polyorganosiloxane resin,
(vi) a gel formed from a silicone and a hydrosilylation compatible solvent wherein said silicone is prepared by the hydrosilylation of a linear hydrogen organopoly-siloxane having two or more hydride functionalities per molecule and an alpha, omega reactive organic molecule possessing two or more reactive functionalities per molecule,
(vii) a gel formed as a reaction product of a vinyl functional hydrido-siloxane in a hydrosilylation-compatible solvent; and
(viii) a gel formed from a polyacrylate siloxane copolymer network swollen in an aqueous and/or non-aqueous swelling liquid; and,
b) the source of silicone oil(s) (c) contains at least one linear, branched or cyclic silicone oil possessing from 2 to 7 silicon atoms and alkyl groups of from 1 to 30 carbon atoms.

3. The process of Claim 2 wherein the source of silicone oil(s) (c) is, or includes, linear, branched or cyclic silicone oil possessing from 3 to 6 silicon atoms and at least one alkyl group of from 3 to 20 carbon atoms.

4. The process of Claim 3 wherein the source of linear, branched or cyclic silicone oil(s) (c) is, or includes, at least one member selected from the group consisting of octamethylcyclotetrasiloxane, decamethylcyclopentasiloxane, dodecamethylcyclohexasiloxane, heptamethylhexyltrisiloxane, heptamethylheptyltrisiloxane, heptamethyloctyltrisiloxane, hexamethyldisiloxane, octamethyltrisiloxane, pentamethylhexyldisiloxane, decamethyltetrasiloxane, dodecamethylpentasiloxane and capryl methicone.

5. The process of Claim 1 wherein the stable, uniform mixture of (a), (b) and (c) contains from 50 to 80 weight percent silicone gel(s), from 5 to 30 weight percent boron nitride powder(s) and from 5 to 30 weight percent silicone fluid(s).

6. The process of Claim 1 wherein the stable, uniform mixture of (a), (b) and (c) contains from 50 to 75 weight percent silicone gel(s) (a), from 10 to 25 weight percent boron nitride powder(s) (b) and from 10 to 20 weight percent silicone oil(s) (c).

7. The process of Claim 1 wherein, in step (A), at least some (a) is combined with at least some (b) to provide a pre-mix of (a) and (b), at least some (c) thereafter being combined with the pre-mix of (a) and (b).

8. The process of Claim 1 wherein, in step (A), at least some (b) is combined with at least some (c) to provide a pre-mix of (b) and (c), at least some (a) thereafter being combined with the premix of (b) and (c).

9. The process of Claim 1 wherein, in step (A), at least some (a) is combined with at least some (c) to provide a pre-mix of (a) and (c), at least some (b) thereafter being combined with the pre- mix of (a) and (c).

## Patentansprüche

1. Verfahren zum Herstellen einer stabilen Bornitridpulver-haltigen Silikongel-Zusammensetzung zur Körperpflege zum Auftragen auf Haut oder Haare, umfassend:
(A) Kombinieren
(a) einer Quelle von Sikongel(en), deren Gehalt an nicht-Silikon-Materialien nicht mehr als 10 Gew.-% beträgt,
(b) einer Quelle von Bornitridpulver(n), und
(c) einer Quelle von Silikonöl(en)
um ein stabiles, einheitliches Gemisch aus (a), (b) und (c) zu erhalten, das 50 bis 80 Gew.-% an Silikongel(en) enthält, wobei der Rest der Zusammensetzung aus Bornitridpulver und Silikonöl gebildet wird; und
(B) Kombinieren des stabilen, einheitlichen Gemischs aus (a), (b) und (c) mit zumindest einem Bestandteil ausgewählt aus der Gruppe bestehend aus anderen Silikonen, einem Tensid, einem Emulgator, einem Lösungsmittel, einem Weichmacher, einem Befeuchter, einem Feuchthaltmittel, einem Pigment, einem Farbstoff, einem Duftstoff, einem Konservierungsmittel, einem Antioxidans, einem Biozid, einem Biostat, einem Antiperspirant, einem Peeling, einem Hormon, einem Enzym, einem Medikament, einer Vitaminsubstanz, einer Hydroxysäure, einem Retinal, einem Retinolderivat, einem Niacinamid, einem Hautaufheller, einem Salz, einem Elektrolyt, einem Alkohol, einem Polyol, einem Mittel zum Brechen und/oder Absorbieren von ultravioletter Strahlung, einem Pflanzenextrakt, einem Peptid, einem Protein, einem organischen Öl, einem Gummi, einem Saccharid, einem Oligosaccharid, einem Polysaccharid, einem Wachs, einem Schichtbildner, einem Verdickungsmittel, einem Partikelfüllstoff, einem Weichmacher, einem Befeuchter, einem Sinnesverstärker, einem Harz und einem optisch aktiven Partikel.

2. Verfahren nach Anspruch 1, wobei:
a) die Quelle von Silikongel(en) (a) zumindest ein Mitglied ausgewählt aus der Gruppe bestehend aus:
(i) einem Gel, das aus einem Silikon und einem Hydrosilylierungs-kompatiblen Lösungsmittel gebildet ist, wobei das Silikon hergestellt wird durch die Hydrosilylierung eines linearen Alkenyl-Polyorganisiloxans und einem Hydridharz,
(ii) einem Gel, das als Reaktionsprodukt eines Epoxy-funktionellen Hydrid-Siloxans gebildet ist, wobei dieses Reaktionsprodukt in einem Epoxid-Gel-bildungskompatiblen Lösungsmittel gebildet wird,
(iii) einem Gel, das aus einem Silikon und einem Hydrosilylierungs-kompatiblen Lösungsmittel gebildet ist, wobei das Silikon hergestellt wird durch die Hydrosilylierung eines linearen Wasserstoff-Polyorganosiloxans und einem Alkenylharz,
(iv) einem Gel, das aus einem Silikon und einem Hydrosilylierungs-kompatiblen Lösungsmittel gebildet ist, wobei das Silikon hergestellt wird durch die Hydrosilylierung eines linearen Wasserstoff-Polyorganosiloxans und einem linearen Polyorganosiloxan,
(v) einem Gel, das aus einem Silikon und einem Hydrosilylierungs-kompatiblen Lösungsmittel gebildet ist, wobei das Silikon hergestellt wird durch die Hydrosilylierung eines linearen Wasserstoff-Polyorganosiloxans und einem Alkenyl-Polyorganosiloxanharz,
(vi) einem Gel, das aus einem Silikon und einem Hydrosilylierungs-kompatiblen Lösungsmittel gebildet ist, wobei das Silikon hergestellt wird durch die Hydrosilylierung eines linearen Wasserstoff-Polyorganosiloxans mit zwei oder mehr Hydrifunktionalitäten pro Molekül und einem alpha, omega-reaktiven organischen Molekül mit zwei oder mehr reaktive Funktionalitäten pro Molekül,
(vii) einem Gel, das als ein Reaktionsprodukt eines Hydrid-funktionellen Siloxan in einem Hydrosilylierungs-kompatiblen Lösungsmittel gebildet wird; und
(viii) einem Gel, das aus einem Polyacrylat-Siloxan-Copolymer-Netz gebildet ist, das in einer wässrigen und/oder nicht-wässrigen Schwell-Lösung geschwollen ist,
enthält; und
b) die Quelle von Silikonöl(en) (c) zumindest ein lineares, verzweigtes oder zyklisches Silikonöl mit 2 bis 7 Siliziumatomen und Alkylengruppen mit 1 bis 30 Kohlenstoffatomen enthält.

3. Verfahren nach Anspruch 2, wobei die Quelle von Silikonöl(en) (c) ein lineares, verzweigtes oder zyklisches Silikonöl mit 3 bis 6 Silikonatomen und zumindest einer Alkylgruppe mit 3 bis 20 Kohlenstoffatomen ist oder umfasst.

4. Verfahren nach Anspruch 3, wobei die Quelle des/der linearen, verzweigten oder zyklischen Silikonöls/-e zumindest eines ausgewählt aus der Gruppe bestehend aus Octamethylcyclotretrasiloxan, Decamethylcyclopentasiloxan, Dodecamethylcyclopentasiloxan, Heptamethylhexyltrisiloxan, Heptamethyltrisiloxan, Heptamethyloctyltrisiloxan, Hexamethyldisiloxan, Octamethyltrisiloxan, Pentamethylhexyldisiloxan, Decamethyltetrasiloxan, Dodecamethylpentasiloxan und Caprylmethikon.

5. Verfahren nach Anspruch 1, wobei das stabile, einheitliche Gemisch aus (a), (b) und (c) 50 bis 80 Gew.-% Silikongel, 5 bis 30 Gew.-% Bornitridpulver und 5 bis 30 Gew.-% Silikonfluid(e) enthält.

6. Verfahren nach Anspruch 1, wobei das stabile, einheitliche Gemisch aus (a), (b) und (c) 50 bis 75 Gew.-% Silikongel(e) (a), 10 bis 25 Gew.-% Bornitridpulver (b) und 10 bis 20 Gew.-% Silikonöl(e) (c) enthält.

7. Verfahren nach Anspruch 1, wobei in Schritt (A), zumindest ein Teil von (a) mit zumindest einem Teil von (b) kombiniert wird, um ein Vorgemisch von (a) und (b) bereitzustellen, wobei danach zumindest ein Teil von (c) mit dem Vorgemisch aus (a) und (b) kombiniert wird.

8. Verfahren nach Anspruch 1, wobei in Schritt (a), zumindest ein Teil von (b) mit zumindest einem Teil von (c) kombiniert wird, um ein Vorgemisch aus (b) und (c) bereitzustellen, und zumindest ein Teil von (a) danach mit dem Vorgemisch aus (b) und (c) kombiniert wird.

9. Verfahren nach Anspruch 1, wobei in Schritt (A), zumindest ein Teil von (a) mit zumindest einem Teil von (c) kombiniert wird, um ein Vorgemisch aus (a) und (c) bereitzustellen, wobei danach zumindest ein Teil von (b) mit dem Vorgemisch aus (a) und (c) kombiniert wird.

## Revendications

1. Procédé de préparation d'une composition de soin personnel stable à gel de silicone contenant de la poudre de nitrure de bore destinée à une application sur la peau ou les cheveux, et comprenant les étapes consistant à :
(A) combiner
(a) une source de gel(s) de silicone ne contenant pas plus de 10 pour cent en poids de celle-ci de matériau(x) de gel sans silicone ;
(b) une source de poudre(s) de nitrure de bore, et
(c) une source d'huile(s) de silicone, et
afin de fournir un mélange stable et uniforme de (a), (b) et (c), lequel contient de 50 à 80 pour cent en poids de gel(s) de silicone, le reste de la composition étant constitué de poudre de nitrure de bore et d'huile de silicone, et
(B) combiner le mélange stable et uniforme de (a), (b) et (c) avec au moins un composant sélectionné parmi le groupe consistant en une autre silicone, un tensio-actif, un émulsifiant, un solvant, un émollient, un hydratant, un humectant, un pigment, un colorant, une fragrance, un conservateur, un antioxydant, un biocide, un biostat, un antiperspirant, un exfoliant, une hormone, une enzyme, un médicament, une substance de vitamine, un acide hydroxy, du rétinal, un dérivé de rétinol, un niacinamide, un agent d'éclaircissement de la peau, du sel, un électrolyte, un alcool, un polyol, un agent de diffusion et/ou d'absorption d'esters pour les rayons ultraviolets, un extrait botanique, un peptide, une protéine, une huile organique, une gomme, un saccharide, un oligosaccharide, un polysaccharide, une cire, un agent filmogène, un agent épaississant, une charge particulaire, un agent plastifiant, un humectant, un agent d'amplification sensorielle, une résine, et une particule active sur le plan optique.

2. Procédé selon la revendication 1, dans lequel :
a) la source de gel(s) de silicone (a) contient au moins un élément sélectionné parmi le groupe consistant en :
(i) un gel formé à partir d'une silicone et d'un solvant compatible avec une hydrosilylation, dans lequel ladite silicone est préparée par l'hydrosilylation d'un polyorganosiloxane à alcényle linéaire et d'une résine d'hydrure ;
(ii) un gel formé comme produit de réaction d'un hydridosiloxane à fonction époxy, ledit produit de réaction étant formé dans un solvant compatible avec une formation de gel époxy ;
(iii) un gel formé à partir d'une silicone et d'un solvant compatible avec une hydrosilylation, dans lequel ladite silicone est préparée par l'hydrosilylation d'un polyorganosiloxane à hydrogène linéaire et d'une résine alcényle ;
(iv) un gel formé à partir d'une silicone et d'un solvant compatible avec une hydrosilylation, dans lequel ladite silicone est préparée par l'hydrosilylation d'un polyorganosiloxane à hydrogène linéaire et d'un polyorganosiloxane à alcényle linéaire ;
(v) un gel formé à partir d'une silicone et d'un solvant compatible avec une hydrosilylation, dans lequel ladite silicone est préparée par l'hydrosilylation d'une résine de polyorganosiloxane à hydrogène et d'une résine de polyorganosiloxane à alcényle ;
(vi) un gel formé à partir d'une silicone et d'un solvant compatible avec une hydrosilylation, dans lequel ladite silicone est préparée par l'hydrosilylation d'un polyorganosiloxane à hydrogène linéaire présentant deux ou plus fonctionnalités hydrure par molécule et une molécule organique réactive alpha, oméga possédant deux ou plus fonctionnalités réactives par molécule ;
(vii) un gel formé comme produit de réaction d'un hydridosiloxane à fonction vinyle dans un solvant compatible avec une hydrosilylation, et
(viii) un gel formé à partir d'un réseau de copolymères de polyacrylate-siloxane gonflé dans un liquide gonflant aqueux et/ou non aqueux, et
b) la source d'huile(s) de silicone (c) contenant au moins une huile de silicone linéaire, ramifiée ou cyclique possédant de 2 à 7 atomes de silicium et des groupes alkyle présentant de 1 à 30 atomes de carbone.

3. Procédé selon la revendication 2, dans lequel la source d'huile(s) de silicone (c) est, ou inclut, une huile de silicone linéaire, ramifiée ou cyclique possédant de 3 à 6 atomes de silicium et au moins un groupe alkyle présentant de 3 à 20 atomes de carbone.

4. Procédé selon la revendication 3, dans lequel la source d'huile(s) de silicone (c) linéaires, ramifiées ou cycliques est, ou inclut, au moins un élément sélectionné parmi le groupe consistant en de l'octaméthylcyclotétrasiloxane, du décaméthylcyclopentasiloxane, du dodécaméthylcyclohexasiloxane, de l'heptaméthylhexyltrisiloxane, de l'heptaméthylheptyltrisiloxane, de l'heptaméthyloctyltrisiloxane, de l'hexaméthyldisiloxane, de l'octaméthyltrisiloxane, du pentaméthylhexyldisiloxane, du décaméthyltétrasiloxane, du dodécaméthylpentasiloxane, et de la capryl-méthicone.

5. Procédé selon la revendication 1, dans lequel le mélange stable et uniforme de (a), (b) et (c) contient de 50 à 80 pour cent en poids de gel(s) de silicone, de 5 à 30 pour cent en poids de poudre(s) de nitrure de bore, et de 5 à 30 pour cent en poids de fluide(s) de silicone.

6. Procédé selon la revendication 1, dans lequel le mélange stable et uniforme de (a), (b) et (c) contient de 50 à 75 pour cent en poids de gel(s) de silicone (a), de 10 à 25 pour cent en poids de poudre(s) de nitrure de bore, et de 10 à 20 pour cent en poids d'huile (s) de silicone.

7. Procédé selon la revendication 1, dans lequel lors de l'étape (A), au moins une partie de (a) est combinée avec au moins une partie de (b) pour fournir un prémélange de (a) et (b), au moins une partie de (c) étant ensuite combinée avec le prémélange de (a) et (b).

8. Procédé selon la revendication 1, dans lequel lors de l'étape (A), au moins une partie de (b) est combinée avec au moins une partie de (c) pour fournir un prémélange de (b) et (c), au moins une partie de (a) étant ensuite combinée avec le prémélange de (b) et (c).

9. Procédé selon la revendication 1, dans lequel lors de l'étape (A), au moins une partie de (a) est combinée avec au moins une partie de (c) pour fournir un prémélange de (a) et (c), au moins une partie de (b) étant ensuite combinée avec le prémélange de (a) et (c).
